# EUROPEAN PATENT SPECIFICATION

(11) **EP 3 270 816 B1**
(45) Date of publication and mention of the grant of the patent: **10.07.2019**
(21) Application number: 15709972.2
(22) Date of filing: 17.03.2015
(51) Int. Cl.: A61B 46/00, A61B 46/20, A61B 34/20, A61B 90/90

(54) **SURGICAL DRAPE FOR PATIENT REGISTRATION AND A REGISTRATION METHOD UTILIZING SUCH SURGICAL DRAPE**
OP-ABDECKTUCH FÜR PATIENTENREGISTRIERUNG UND REGISTRIERUNGSVERFAHREN MIT SOLCH EINEM OP-ABDECKTUCH
CHAMP OPÉRATOIRE POUR L'ENREGISTREMENT D'UN PATIENT ET UN PROCÉDÉ D'ENREGISTREMENT UTILISANT UN TEL CHAMP OPÉRATOIRE

(43) Date of publication of application: 24.01.2018
(73) Proprietor: Brainlab AG, 81829 München (DE)
(72) Inventor: HEINDL, Nadja, 80331 Munich (DE); MEZGER, Uli, 85551 Heimstetten (DE); RODAS, Conrad, Longmont, Colorado 80503 (US)
(74) Representative: SSM Sandmair
(86) International application number: PCT/EP2015/055558
(87) International publication number: WO 2016/146173

(56) References cited:
- EP-A2- 0 166 124
- WO-A1-98/08457
- WO-A1-2013/055707
- WO-A2-2013/119801
- US-A1- 2007 235 038
- US-A1- 2012 167 896
- US-A1- 2012 298 115
- US-A1- 2013 133 670

## Description

The present invention relates to a surgical drape for separating a sterile region from an unsterile region during surgery and a method of utilizing such surgical drape during patient registration.

In medical procedures incorporating image-guided surgery (IGS), is desirable to know the position of a specific anatomical structure that can be seen in the medical image of the body of a patient who is to be treated, with respect to the actual patient's body. It is likewise desirable to know the relative position of medical equipment such as medical instruments and a specific anatomic structure that is shown in a medical image of the body of the patient. For both reasons, patient registration procedures have to be performed by means of which the actual body is registered to the at least one medical image. This will allow a medical navigation system to display the position of the anatomical structure relative to the surgical instrument to medical personnel on a monitor.

However, such registration procedures must not compromise sterility of the workplace. Conventional registration methods are therefore performed preoperatively and prior to draping of the patient. Registration cannot be repeated, for example to restore an initial registration intra-operatively, after the patient has been draped, since anatomical landmarks, structures and artificial fiducials have also been covered by the drape and are therefore inaccessible. WO 2005/002456 A1 suggests to provide a surgical drape having covering elements for reference means that allowed to track the reference means intra-operatively. This, however, does not allow for re-registering the patient intra-operatively, as well.

Document EP 0 166 124 discloses a surgical drape according to the preamble of appended claim 1.

Documents WO 98/08457 and US 2012/0298115 disclose alternative surgical drapes comprising an impervious section, an incision section and a transparent section.

The present invention provides a surgical drape and a method of registering a patient's body part, that allow to register a patient's body part intra-operatively after it has been covered by a surgical drape prior to surgery.

The inventive surgical drape and the inventive method of registering a patient's body part are defined by the appended independent claims. Advantages, advantageous futures, advantageous embodiments and advantageous aspects of the present invention are disclosed in the following and contained in the subject-matter of the dependent claims. Different advantageous futures can be combined in accordance with the invention wherever technically expedient and feasible. Specifically, a feature of one embodiment which has the same or a similar function to another feature of another embodiment can be exchanged with said other feature. A feature of one embodiment which adds an additional function to another embodiment can in particular be added to said other embodiment.

The surgical drape according to the present invention comprises:
- an impervious section (2) having absorbing properties;
- an incision section (3) comprising an incise foil; and
- a transparent section (4)
adapted to allow a visual observation through the surgical drape (1) of a substantial part of a patient for registration purposes that is covered by the surgical drape (1), characterized in that
the incision section (3) and the transparent section (4) lie adjacent to each other and the transparent section (4) is flanked by the impervious section (2).

The inventive surgical drape comprises an impervious section having absorbing properties which is already known from prior art surgical drapes and which has a layer configured to absorb body fluids, and a further layer that is configured to provide an impervious barrier preventing any substance to penetrate the impervious section of the surgical drape. For example, the impervious layer may be a layer of the impervious section adjacent to an unsterile section underneath the drape covering the patient, whereas the absorbing section may face towards the sterile workplace. Moreover, the inventive surgical drape comprises an incision section having an incision foil which may have adhesive properties on one side to stick the incision section down on the patient's skin right next to the incision area. Additionally to the impervious section and the incision section, the inventive surgical drape further comprises a transparent section trough which medical personnel can see the patient and medical equipment covered by the drape to such an extend so that a registration procedure can be performed on a body part of the patient. For this purpose, the transparent section allows a surgeon to immediately see any anatomical or artificial landmarks the position of which has to be determined for registration.

The transparent section may therefore be sized such that substantially a whole body part to be registered, particularly a structurally stable body part such as a head, a thigh, a lower leg, an upper arm, a forearm or a pelvis covered by the surgical drape (1) can be visually observed.

According to this aspect, the size of the transparent section corresponds to the size of the body part to be registered. For surgery performed on a human head, the transparent section may therefore be sized such that the whole head can be observed through the transparent section, whereas for knee surgery, for example, the transparent section can be sized such that a substantial part of the thigh as well as a substantial part of the lower leg can be covered by and observed through the transparent section of the surgical drape.

The same applies to the shape of the transparent section which can correspond to the shape of the patient's body part to be treated. For example, a surgical drape used for head surgery can have a transparent section with a round or an oval shape, whereas a drape for operations performed on a human leg may have a transparent section with a longitudinal shape.

For example, the size the transparent section can amount to more than 20 % of the overall size of the surgical drape or even to more than 30 % of the overall size of the surgical drape.

The transparent section may be positioned relative to the incision section of the surgical drape in any expedient manner. The surgical drape has an incision section and a transparent section which lie adjacent to each other. The inventive surgical drape can have an incision section surrounded by a transparent section which in turn forms a window within the surgical drape.

It is evident from the description that the transparent section of the inventive surgical drape may have any form and size that appears to be expedient for a specific surgical procedure to be performed.

Further, the surgical drape can be pre-assembled, for example by gluing the impervious section, the incision section and the transparent section together. Moreover, the surgical drape can have the form of a sheet that extends in two dimensions without having any bulge or pouch.

Another example of the inventive surgical drape comprises fixation means that releasably hold together a compacted area of the surgical drape's transparent section, for example a folded, pleated or rolled area. This will allow, medical personnel to increase the size of the transparent section up to a size that is deemed to be appropriate for a specific procedure performed. For example, the transparent section of the surgical drape can be provided with Velcro-straps or adhesive straps that hold a certain portion of the transparent section together, and may be released in case a lager transparent section is needed.

A further aspect of the present invention relates to a method of registering a patient's body part with a pre-acquired image dataset of the body part, comprising the following steps:
- providing a surgical drape comprising a transparent section, particularly a surgical drape according to claim 1;
- covering the patient's body part with the surgical drape so as to provide a sterile workplace separated from an unsterile area by the surgical drape;
- use the transparent section of the surgical drape to visually identify the patient's body part covered by the surgical drape; and
- approach and register the patient's body part with a registration instrument, particularly a pointer instrument.

As already explained further above, the inventive registering method makes use of a transparent section of a surgical drape that allows any medical personal to visually observe a patient's body part and medical equipment through transparent section of the surgical drape for registration purposes.

Again, it is noted that for registration purposes, it is necessary to see large parts of the patient's body together with natural landmarks of artificial fiducials attached to the body part through the drape, so that these landmarks or fiducials can be approached and palpated fast and easily with a registration instrument.

The inventive method can be performed with an unsterile registration instrument held and moved by a user within an unsterile area. In other words, landmarks or fiducials are palpated with an instrument held under the surgical drape, wherein the user can see the instrument through the transparent section of the surgical drape. In case the instrument is tracked via optical tracking cameras, the surgical drape's transparent section has to be translucent for the wavelengths used by the tracking cameras.

In the alternative, the inventive method can also be performed with a sterile registration instrument held and moved within the sterile workplace, i. e. above the surgical drape. In this case it is only necessary to see substantial parts of the patient's body part together with the landmarks or fiducials through the transparent section of the drape, so that the registration procedure can be performed fast and easily.

The inventive method can also comprise a surface matching procedure for which the surgeon sweeps the tip of a registration instrument over the patient's skin so as to determine its form and position. On the other hand, the registration can be performed by palpating predetermined natural landmarks or artificial fiducials for matching them to corresponding landmarks or fiducials, respectively, which can be identified in an image or an image data set of the patient.

With the large transparent section providing the possibility to observe whole body parts of the patient through the surgical drape, it is evident that the registration procedure can be performed prior to surgery. In this case it is possible to attach artificial fiducials onto the patient's skin before the patient is covered with the surgical drape.

With a surgical drape having a size-adjustable transparent section that can be used for a multitude of surgical procedures requiring different sizes of the transparent section, medical personnel can adjust the size of the transparent section by at least partially releasing the fixation means holding together a compacted area of the transparent section.

In the following, the invention is described with reference to the figures which represent preferred embodiments of the invention without limiting the invention to the specific features shown in the figures.
- Figure 1: shows a first embodiment of the inventive surgical drape;
- Figure 2: shows a second embodiment of the inventive surgical drape;
- Figure 3: shows a third embodiment of the inventive surgical drape; and
- Figure 4: illustrates a first embodiment of the inventive registration method.

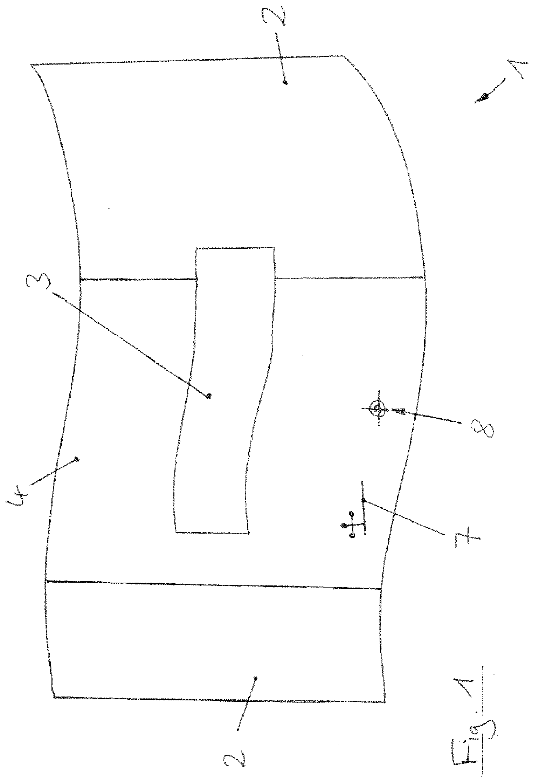

The surgical drape 1 shown in figure 1 comprises a transparent section 4 that extends over the whole width of the surgical drape 1 and is flanked on both sides by impervious sections 2. The surgical drape 1 further comprises an incision section 3 that can be adhered to the skin of a patient to be treated. Figure 1 further shows that the transparent section 4 provides a large area in the vicinity of the incision section 3 that enables medical personnel to visually observe not only the incision area but also large parts of the patient's body. Figure 1 illustrates that a pointer instrument 7 and an artificial fiducial 8 attached to the patient's skin can be seen through transparent section 4 of the surgical drape 1.

It is important to note that incision section 3 does not necessarily have to be entirely surrounded by the transparent section 4 but may also, at least partially, border directly to the impervious section 2 of the surgical drape 1. Moreover, it is to be noted that the size and shape of the impervious sections 2, the incision section 3 and the transparent section 4 as shown in the Figures are arbitrary and that these sections may have any form or size that appears to be suitable for a specific surgical procedure the surgical drape is designed for.

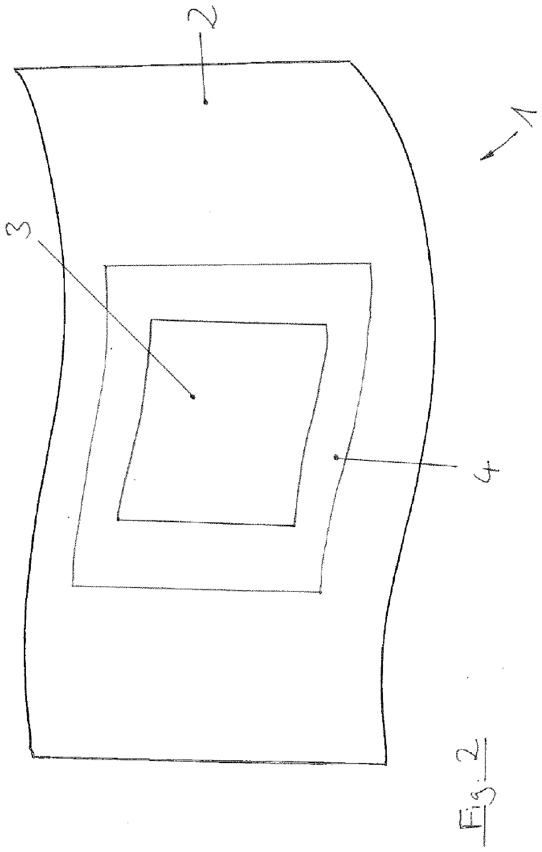

Figure 2 shows another embodiment of the inventive surgical drape 1 having an incision section 3 entirely surrounded by a transparent section 4, which in turn is embedded in an impervious section 2.

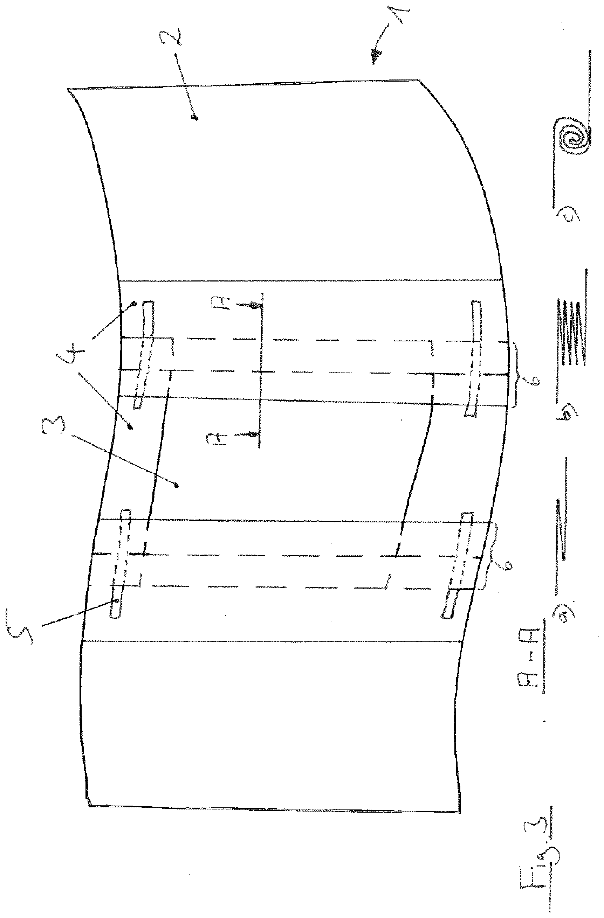

Figure 3 shows a further embodiment of the inventive surgical drape 1 with the transparent section 4 extending over the whole width of the surgical drape 1. The transparent section 4 comprises four adhesive straps that hold together two pleated areas 6. Example b) of Figure 3 shows a cross-sectional view along line A-A through one of the pleated areas 6. The folding edges of the pleated areas 6 as well as the edges of the adhesive straps 5 and the edges of the incision section 3 that are covered by the uppermost layer of the transparent section 4 are shown in broken lines. It is to be noted that the compacted areas 6 can also be formed by a mere double-fold or by coiling up parts of the transparent section, as it is shown by examples a) and c) of the cross-sectional view A-A. The compacted areas 6 of the surgical drape 1 shown in Figure 3 allows medical personnel to adjust the size of the transparent section 4 according to a specific surgical procedure to be performed.

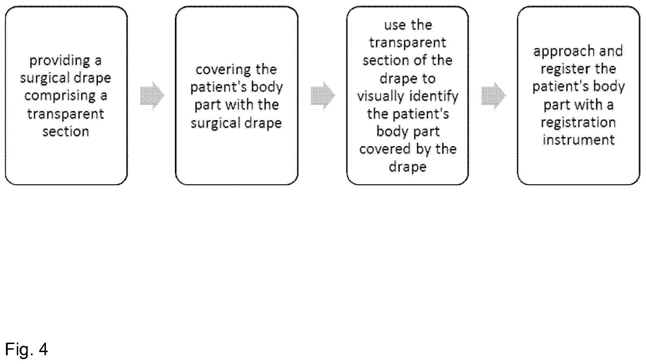

One embodiment of the inventive registration method is illustrated in Figure 4. A surgical drape, for example a drape 1 as described herein, having a transparent section 4 is provided and used to cover a patient's body part a surgical procedure is planned to be performed on. After the patient's body part has been draped it can still be seen through the transparent section 4 of the surgical drape 1 along with natural landmarks and artificial fiducials 8 that might be attached to the patient's skin before it was covered by the drape 1. This enables medical personnel to use a registration instrument such as a pointer instrument 7 to palpate the landmarks or fiducials fast and easily, or simply sweeping the tip of the instrument 7 across the patient's skin for surface matching. This can either be done with an unsterile instrument 7 moved underneath the drape 1 or with a sterile instrument moved above the drape 1 within the sterile working place.

## Claims

1. A surgical drape (1) comprising:
- an impervious section (2) having absorbing properties;
- an incision section (3) comprising an incise foil; and
- a transparent section (4)
adapted to allow a visual observation through the surgical drape (1) of a substantial part of a patient for registration purposes that is covered by the surgical drape (1),
**characterized in that**
the incision section (3) and the transparent section (4) lie adjacent to each other and the transparent section (4) is flanked by the impervious section (2).

2. The surgical drape according to claim 1, wherein the transparent section (4) is sized such that substantially a whole body part, for example a structurally stable body part such as a head, a thigh, a lower leg, an upper arm, a forearm or a pelvis covered by the surgical drape (1) can be visually observed.

3. The surgical drape according to claim 1 or claim 2, wherein the size of the transparent section (4) is more than 20%, particularly more than 30% of the overall size of the surgical drape (1).

4. The surgical drape according to any one of claims 1 to 3, wherein the transparent section (4) is sized and/or shaped corresponding to the size and/or shape of the patient's body part to be treated, and particularly covers at least the patient's body part to be observed.

5. The surgical drape according to any one of claims 1 to 4, wherein the transparent section (4) surrounds the incision section (3).

6. The surgical drape according to any one of claims 1 to 5, wherein surgical drape (1) is pre-assembled and/or has the form of a 2-dimensional plane.

7. The surgical drape according to any one of claims 1 to 6, further comprising fixation means (5) releasably holding together a compacted area (6) of the surgical drapes transparent section (4), for example a folded, pleated or rolled area.

8. A method of registering a patient's body part with a pre-acquired image dataset of the body part, comprising the following steps:
- providing a surgical drape (1) comprising a transparent section (4), for example a surgical drape (1) according to one of the claims 1 to 7;
- covering the patient's body part with the surgical drape (1) so as to provide a sterile workplace separated from an unsterile area by the surgical drape (1);
- use the transparent section (4) of the surgical drape (1) to visually identify the patient's body part covered by the surgical drape (1); and
- approach and register the patient's body part with a registration instrument (7), for example a pointer instrument.

9. The method of claim 8, wherein an unsterilized registration instrument (7) is used within the unsterile area to register the patient's body part.

10. The method of claim 8, wherein a sterile registration instrument (7) is used within the sterile workplace to register the patient's body part.

11. The method of claim 9 or claim 10, wherein registering comprises palpating and/or sweeping the patient's body part with the registration instrument (7) and/or palpating artificial fiducials (8) attached to the patient's body part.

12. The method of any one of claims 8 to 11, further comprising the step of attaching artificial fiducials (8) onto the patient's body part prior to covering the patient with the surgical drape (1).

13. The method of any one of claims 8 to 12, wherein the registration of the patient's body part is an initial registration performed prior to surgery.

14. The method of any one of claims 8 to 13, further comprising the step of adjusting the size of the transparent section (4) by releasing the fixation means (5) of the surgical drape (1) according to claim 7.

## Patentansprüche

1. Chirurgisches Abdecktuch (1) mit:
- einem undurchlässigen Abschnitt (2) mit aufsaugenden Eigenschaften;
- einem Inzisionsabschnitt (3) mit einer Inzisionsfolie; und
- einem transparenten Abschnitt (4), der dazu ausgestaltet ist, durch das chirurgische Abdecktuch (1) hindurch eine visuelle Untersuchung eines wesentlichen Teils eines Patienten zu Registrierungszwecken zu ermöglichen, welcher durch das chirurgische Abdecktuch (1) abgedeckt ist, **dadurch gekennzeichnet, dass**
der Inzisionsabschnitt (3) und der transparente Abschnitt (4) aneinander angrenzen und der transparente Abschnitt durch den undurchlässigen Abschnitt (2) flankiert wird.

2. Chirurgisches Abdecktuch gemäß Anspruch 1, wobei der transparente Abschnitt (4) so bemessen ist, dass im Wesentlichen ein ganzer Körperteil, beispielsweise ein strukturell fester Körperteil wie etwa ein Kopf, ein Oberschenkel, ein Unterschenkel, ein Oberarm, ein Unterarm oder ein Becken, welcher durch das chirurgische Abdecktuch (1) abgedeckt ist, visuell untersucht werden kann.

3. Chirurgisches Abdecktuch gemäß Anspruch 1 oder Anspruch 2, wobei die Größe des transparenten Abschnitts (4) mehr als 20%, insbesondere mehr als 30% der Gesamtgröße des chirurgischen Abdecktuchs (1) beträgt.

4. Chirurgisches Abdecktuch gemäß einem der Ansprüche 1 bis 3, wobei der transparente Abschnitt (4) in Größe und/oder Form entsprechend der Größe und/oder Form des zu behandelnden Körperteils des Patienten bemessen ist, und insbesondere zumindest den zu untersuchenden Körperteil des Patienten abdeckt.

5. Chirurgisches Abdecktuch gemäß einem der Ansprüche 1 bis 4, wobei der transparente Abschnitt (4) den Inzisionsabschnitt (3) umgibt.

6. Chirurgisches Abdecktuch gemäß einem der Ansprüche 1 bis 5, wobei das chirurgische Abdecktuch (1) vorgefertigt ist und/oder die Form einer 2-dimensionalen Ebene hat.

7. Chirurgisches Abdecktuch gemäß einem der Ansprüche 1 bis 6, mit ferner Befestigungsmitteln (5), welche einen komprimierten Bereich (6) des transparenten Abschnitts (4) des chirurgischen Abdecktuchs lösbar zusammenhalten, beispielsweise einen gefalteten, plissierten oder gerollten Bereich.

8. Verfahren zum Registrieren eines Körperteils eines Patienten mit einem zuvor erlangten Bilddatensatz des Körperteils, umfassend die folgenden Schritte:
- Bereitstellen eines chirurgischen Abdecktuchs (1) mit einem transparenten Abschnitt (4), beispielsweise einem chirurgischen Abdecktuch (1) gemäß einem der Ansprüche 1 bis 7;
- Abdecken des Körperteils des Patienten mit dem chirurgischen Abdecktuch (1), um einen sterilen Arbeitsplatz zu schaffen, der von einem unsterilen Bereich mittels des chirurgischen Abdecktuchs (1) abgetrennt wird;
- Verwenden des transparenten Abschnitts (4) des chirurgischen Abdecktuchs (1) zur visuellen Identifizierung des durch das chirurgische Abdecktuch (1) abgedeckten Körperteils des Patienten; und
- Annähern an und Registrieren des Körperteils des Patienten mit einem Registrierungsinstrument (7) beispielsweise einem Zeigeinstrument.

9. Verfahren gemäß Anspruch 8, wobei ein unsterilisiertes Registrierungsinstrument (7) innerhalb des unsterilen Bereichs verwendet wird, um den Körperteil des Patienten zu registrieren.

10. Verfahren gemäß Anspruch 8, wobei ein steriles Registrierungsinstrument innerhalb des sterilen Arbeitsplatzes verwendet wird, um dem Körperteil des Patienten zu Registrieren.

11. Verfahren gemäß Anspruch 9 oder Anspruch 10, wobei das Registrieren ein Abtasten und/oder Überstreichen des Körperteils des Patienten mit dem Registrierungsinstrument (7) und/oder Abtasten künstlicher, am Körperteil des Patienten befestigter Bezugsmarkierungen (8) umfasst.

12. Verfahren gemäß einem der Ansprüche 8 bis 11, mit ferner dem Schritt des Anbringens künstlicher Bezugsmarkierungen (8) auf den Körperteil des Patienten, bevor der Patient mit dem chirurgischen Abdecktuch (1) abgedeckt wird.

13. Verfahren nach einem der Ansprüche 8 bis 12, wobei die Registrierung des Körperteils des Patienten eine initiale Registrierung ist, die vor einem chirurgischen Eingriff durchgeführt wird.

14. Verfahren gemäß einem der Ansprüche 8 bis 13, mit ferner dem Schritt des Einstellens der Größe des transparenten Abschnitts (4) durch Lösen der Befestigungsmittel (5) des chirurgischen Abdecktuchs (1) gemäß Anspruch 7.

## Revendications

1. Drap chirurgical (1) comprenant :
- une section imperméable (2) présentant des propriétés d'absorption ;
- une section d'incision (3) comprenant une feuille d'incision ; et
- une section transparente (4)
conçue pour permettre une observation visuelle à travers le drap chirurgical (1) d'une partie importante d'un patient à des fins d'enregistrement qui est couverte par le drap chirurgical (1), **caractérisé en ce que**
la section d'incision (3) et la section transparente (4) sont situées de manière adjacente l'une à l'autre et la section transparente (4) est flanquée de la section imperméable (2).

2. Drap chirurgical selon la revendication 1, dans lequel la section transparente (4) est dimensionnée de manière que sensiblement tout une partie de corps, par exemple une partie de corps structurellement stable telle qu'une tête, une cuisse, la partie inférieure d'une jambe, un arrière-bras, un avant-bras ou un pelvis recouvert du drap chirurgical (1) peut être observée.

3. Drap chirurgical selon la revendication 1 ou la revendication 2, dans lequel la taille de la section transparente (4) est de plus de 20 %, en particulier plus de 30 % de la taille globale du drap chirurgical (1).

4. Drap chirurgical selon l'une quelconque des revendications 1 à 3, dans lequel la section transparente (4) présente une dimension et/ou une forme correspondant à la dimension et/ou la forme de la partie du corps du patient à traiter, et en particulier recouvre au moins la partie du corps du patient à observer.

5. Drap chirurgical selon l'une quelconque des revendications 1 à 4, dans lequel la section transparente (4) entoure la section d'incision (3).

6. Drap chirurgical selon l'une quelconque des revendications 1 à 5, le drap chirurgical (1) étant préassemblé et/ou présentant la forme d'un plan en 2 dimensions.

7. Drap chirurgical selon l'une quelconque des revendications 1 à 6, comprenant en outre un moyen de fixation (5) maintenant ensemble de manière amovible une zone de compactage (6) de la section transparente du drap chirurgical (4), par exemple une zone pliée, plissée ou roulée.

8. Procédé d'enregistrement d'une partie du corps d'un patient avec un ensemble de données d'image pré-acquise de la partie du corps, comprenant les étapes suivantes :
- fourniture d'un drap chirurgical (1) comprenant une section transparente (4), par exemple un drap chirurgical (1) selon l'une des revendications 1 à 7 ;
- recouvrement de la partie du corps du patient avec le drap chirurgical (1) de manière à créer un lieu de travail stérile séparé d'une zone non stérile par le drap chirurgical (1) ;
- utilisation de la section transparente (4) du drap chirurgical (1) pour identifier visuellement la partie du corps du patient recouverte par le drap chirurgical (1) ; et
- s'approcher de la partie du corps du patient et enregistrer celle-ci avec un instrument d'enregistrement (7), par exemple un instrument de pointage.

9. Procédé selon la revendication 8, dans lequel un instrument d'enregistrement non stérilisé (7) est utilisé dans une zone non stérile pour enregistrer la partie du corps du patient.

10. Procédé selon la revendication 8, dans lequel un instrument d'enregistrement stérile (7) est utilisé sur un lieu de travail stérile pour enregistrer la partie du corps du patient.

11. Procédé selon la revendication 9 ou la revendication 10, dans lequel l'enregistrement comprend la palpation et/ou le balayage de la partie du corps du patient avec l'instrument d'enregistrement (7) et/ou la palpation de repères d'alignement artificiels (8) fixés à la partie du corps du patient.

12. Procédé selon l'une quelconque des revendications 8 à 11, comprenant en outre l'étape de fixation de repères d'alignement artificiels (8) sur la partie du corps du patient avant de recouvrir le patient avec le drap chirurgical (1).

13. Procédé selon l'une quelconque des revendications 8 à 12, dans lequel l'enregistrement de la partie du corps du patient est un enregistrement initial effectué avant la chirurgie.

14. Procédé selon l'une quelconque des revendications 8 à 13, comprenant en outre l'étape d'ajustement de la dimension de la section transparente (4) par le relâchement du moyen de fixation (5) du drap chirurgical (1) selon la revendication 7.
